(19) Europäisches Patentamt
European Patent Office
Office européen des brevets

(11) Publication number: **0 007 717**
**B1**

(12) # EUROPEAN PATENT SPECIFICATION

(45) Date of publication of patent specification: **11.08.82**

(51) Int. Cl.$^3$: **C 07 D 498/04,**
A 61 K 31/42

(21) Application number: **79301284.0**

(22) Date of filing: **02.07.79**

(54) **Clavulanic acid derivatives, a process for their preparation, their use, and pharmaceutical compositions containing them.**

(30) Priority: **29.07.78 GB 3162478**

(43) Date of publication of application:
**06.02.80 Bulletin 80/3**

(45) Publication of the grant of the patent:
**11.08.82 Bulletin 82/32**

(84) Designated Contracting States:
**BE CH DE FR GB IT NL SE**

(56) References cited:
**DE - A - 2 646 003**
**DE - A - 2 817 085**

(73) Proprietor: **BEECHAM GROUP PLC**
**Beecham House Great West Road**
**Brentford Middlesex (GB)**

(72) Inventor: **Stirling, Irene**
**80 Woodcrest Walk**
**Reigate, Surrey (GB)**
Inventor: **Clarke, Brian Peter**
**"Coneybury" Drive Spur Forest Drive**
**Kingswood, Surrey (GB)**

(74) Representative: **Hesketh, Alan, Dr. et al,**
**European Patent Attorney Beecham**
**Pharmaceuticals Great Burgh Yew Tree Bottom**
**Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Clavulanic acid derivatives, a process for their preparation, their use, and pharmaceutical compositions containing them

The present invention relates to β-lactam anti-bacterial agents, to the process for their preparation and to compositions containing them.

British Patent Application No. 16764/77 (see also U.S. Serial No. 896441, Belgian Patent No. 187034 and West German Offenlegunsschrift No. P2817085.6) discloses *inter alia* the compounds of the formula (I):

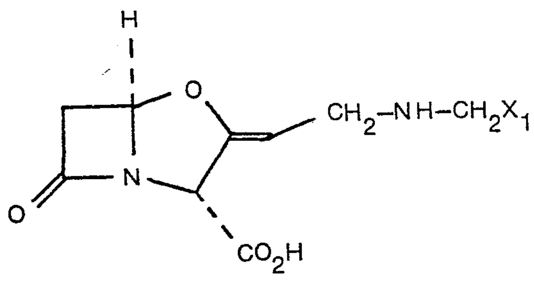

(I)

and esters thereof wherein $X_1$ is a hydrogen atom, an alkyl group of up to 5 carbon atoms, an alkenyl group of up to 6 carbon atoms, a hydroxy alkyl group of up to 5 carbon atoms or an optionally substituted phenyl group. Such compounds were described as antibacterial agents and β-lactamase inhibitors.

It has now been discovered that a crystalline secondary amine can be prepared that is a β-lactamase inhibitor that enhances the effectiveness of penicillins or cephalosporins and which also has antibacterial properties in its own right and which is advantageously water soluble.

The present invention provides the compound of the formula (II):

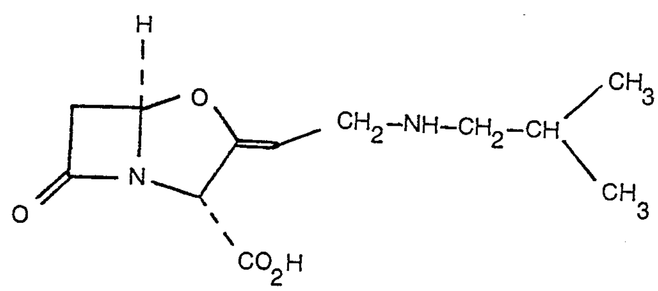

(II)

The compound of the formula (II) per se exists in the form of a zwitterion, that is it may be represented as shown in formula (IIa):

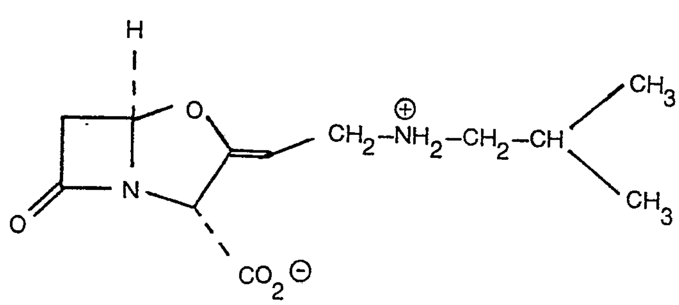

(IIa)

This zwitterionic compound is favoured in view of its crystalline form, stability and greater solubility than previously prepared β-lactamase inhibitory amines such as those within the formula (I).

As has been previously indicated we prefer to prepare and use the crystalline zwitterionic compound of formula (II). However, esters of the compound of the formula (II) also form part of this invention, for example as the free base or as the acid additional salt since such compounds may also be used to enhance the effectiveness of penicillins or cephalosporins.

Certain suitable esters of the compounds of the formula (II) — include those of the formula (III) and (IV):

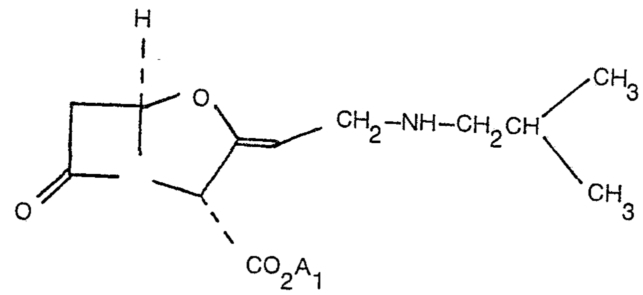

(III)

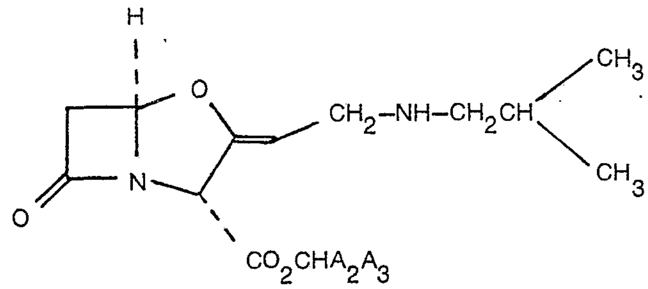

(IV)

wherein $A_1$ is an alkyl group of 1—6 carbon atoms optionally substituted by an alkoxyl or acyloxyl group of 1—7 carbon atoms; $A_2$ is an alkenyl or alkynyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

Suitable esters of the compound of the formula (II) include the methyl, ethyl, n-propyl, n-butyl, allyl, $CH_2—C{\equiv}CH$, methoxymethyl, acetoxymethyl, propionoxymethyl, pivaloyloxymethyl, ethoxycarbonyloxymethyl, methoxycarbonyloxyethyl, ethoxycarbonyloxyethyl, dimethoxyphthalidyl, benzyl, methoxybenzyl, ethoxybenzyl, nitrobenzyl, chlorobenzyl or the like ester such as the 2-methylallyl ester.

Certain favoured groups $A_1$ include the methyl, methoxymethyl, acetoxymethyl, acetoxyethyl, phthalidyl, ethoxycarbonyloxymethyl, $\alpha$-ethoxycarbonyloxyethyl and the like groups.

Certain favoured groups $A_2$ include the phenyl and 4-methoxyphenyl groups. A particularly favoured moiety $A_3$ is the hydrogen atom.

Certain other favoured values for $A_1$ include those of the sub-formulae (c), (d) and (e):

$$—CHA_5—OA_6 \qquad (c)$$

$$—CHA_5—COA_6 \qquad (d)$$

$$—CHA_5—CO_2A_6 \qquad (e)$$

wherein $A_5$ is a hydrogen atom or a methyl group and $A_6$ is an alkyl group of up to 4 carbon atoms or a phenyl or benzyl group either of which may be substituted by one or two alkyl or alkoxyl groups of up to 3 carbon atoms or by a fluorine, chlorine or bromine atom or a nitro group; or $A_5$ is joined to $A_6$ to form the residue of an unsubstituted saturated 5- or 6-membered heterocyclic ring or an ortho-phenylene group which may be substituted by one or two alkyl or alkoxyl groups of up to 3 carbon atoms or by a fluorine, chlorine or bromine atom or nitro group.

An apt acyclic value for the sub-group of the formula (c) is $—CH_2—OA_6$.

An apt acyclic value for the sub-group of the formula (d) is $—CH_2—CO—A_6$.

An apt acyclic value for the sub-group of the formula (e) is $—CH_2—CO_2A_6$.

A further apt acyclic value for the sub-group of the formula (3) is $—CH(CH_3)—CO_2A_6$.

Favoured values for $A_6$ in the preceding acyclic moieties include the methyl, ethyl, propyl, butyl, phenyl and benzyl groups.

Apt cyclic values for the sub-group of the formula (c) include the tetrahydropyranyl and tetrahydrofuranyl groups.

Esters of the compounds of the formula, (II) such as those of the compounds of the formulae (III) or (IV) may be presented in the form of their acid addition salts if desired. The acid used to form the salt will most suitably be pharmaceutically acceptable, but non-pharmaceutically acceptable acid addition salts are also envisaged, for example as intermediates in the preparation of the pharmaceutically acceptable salts by ion exchange. Suitable pharmaceutically acceptable acid additions salts include

those of inorganic and organic acids, such as hydrochloric, phosphoric, sulphuric, methanesulphonic, toluenesulphonic, citric, malic, acetic, lactic, tartaric, propionic, succinic or the like acid.

Most suitably the acid addition salt is provided as a solid and preferably as a crystalline solid.

Compounds of this invention wherein crystalline form may be solvated, for example hydrated.

The present invention provides a pharmaceutical composition which comprises a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form. The zwitterionic compounds of this invention are particularly suitable for use in such compositions.

The injectable solution of the compound of this invention may be made up in a sterile pyrogen-free liquid such as water, aqueous ethanol or the like.

Compounds of this invention when in highly pure crystalline form tend to have higher aqueous solubilities than those of our previously prepared pure crystalline 9-amino-deoxyclavulanic acid derivatives such as 9-N-benzylaminodeoxyclavulanic acid. This means that the compounds are easier to administer than the previously known compounds. In this circumstance it is often convenient to administer the solution by intra-muscular injection which is less complicated than intravenous administration.

This invention also provides the use of the compound of the formula (II) for preparing injectable aqueous solutions. Such solutions may be prepared by dissolving the sterile compound of the formula (II) in sterile water. Suitably this water is "Water for Injection BP" or the equivalent and may contain electrolytes to render it isotonic.

A particularly suitable injectable aqueous solution of this invention is one which contains not less than 15% w/w of the compound of the formula (II), favourably not less than 20% w/w of the compound of the formula (II) and preferably not less than 25% w/w of the compound of the formula (II).

Unit dose compositions comprising a compound of this invention adapted for oral administration form a further suitable composition aspect of this invention. However, orally administrable forms are generally less favoured than injectable forms owing to the relatively poor absorption of the compounds from the gastro-intestinal tract. Despite this orally administrable compositions are of use as a synergistically effective blood level can be expected at high doses and at lower doses such compositions may be used to treat infections localised in the gastro-intestinal tract.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compounds of the formula (II) may be present in the composition as sole therapeutic agent or they may be present together with other therapeutic agents such as penicillin or cephalosporin. Considerable advantages accrue from the inclusion of a penicillin or cephalosporin since the resulting composition shows enhanced effectiveness (synergy).

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin and other known penicillins including prodrugs therefor such as their in-vivo hydrolysable esters such as the acetoxymethyl, pivaloyloxymethyl, $\alpha$-ethoxycarbonyloxyethyl or phthalidyl esters of ampicillin, benzylpenicillin or amoxycillin, and aldehyde or ketone adducts of penicillins containing a 6-$\alpha$-aminoacetamide side chain (such as hetacillin, metampicillin and analogous derivatives of amoxycillin) or $\alpha$-esters of carbenicillin or ticarcillin such as their phenyl or indanyl $\alpha$-esters.

Suitable cephalosporins for inclusion in the compositions of this invention include cefatrizine, cephaloridine, cephalothin, cefazolin, cephalexin, cephacetrile, cephamandole nafate, cephapirin, cepradine, 4-hydroxycephalexin, cefaparole, cephaloglycin, and other known cephalosporins or prodrugs thereof.

Such compounds are frequently used in the form of a salt or hydrate or the like.

Naturally if the penicillin or cephalosporin present in the composition is not suitable for oral administration then the composition will be adapted for parenteral administration. As previously indicated such injectable or infusable compositions are preferred.

Highly favoured penicillins for use in the compositions of this invention include ampicillin,

amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin. Such cephalosporins may be used as a pharmaceutically acceptable salt, for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent). Orally administrable compositions containing a compound of the invention will normally contain relatively more synergist than corresponding injectable compositions, for example the ratio in an oral composition may be from about 3:1 to about 1:1 whereas a corresponding injectable composition may contain a ratio of about 1:1 to about 1:3 (compound of the invention: penicillin or cephalosporin).

The total quantity of a compound of the invention in any unit dosage will normally be between 25 and 1000 mg and will usually be between 50 and 500 mg, for example about 62.5, 100, 125, 150, 200 or 250 mg.

Compositions of this invention may be used for the treatment of infections inter alia, the respiratory tract, the urinary tract and soft tissues in humans and mastitis in cattle.

Normally between 50 and 1000 mg of the compounds of the invention will be administered each day of treatment but more usually between 100 and 750 mg of the compounds of the invention will be administered per day, for example as 1—6 doses, more usually as 2, 3 or 4 doses.

The penicillin or cephalosporin in the synergistic composition of this invention will normally be present at approximately the amount at which it is conveniently used which will usually be expected to be from about 62.5 to 1000 mg per dose, more usually about 125, 250 or 500 mg per dose.

One particularly favoured composition of this invention will contain from 150 to 1000 mg of amoxycillin as the trihydrate or sodium salt and from 25 to 500 mg of a compound of this invention.

Most suitably this form of composition will contain the compound of the formula (II).

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillin hydrochloride bacampicillin hydrochloride or talampicillin hydrochloride. Most suitably this form of the composition will contain a compound of the formula (II).

Most suitably the preceding compositions will contain from 200 to 700 mg of the penicillin component. Most suitably the preceding composition will comprise from 50 to 250 mg of a compound of the formula (II) in crystalline form.

Such compositions may be adapted for oral or parenteral use except when containing in-vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the formula (II) preferably in crystalline form. Such compositions containing di-salts of carbenicillin and ticarcillin will be adapted for parenteral administration.

Commonly the infection treated will be due to a strain of *Staphylococcus aureus, Klebsiella aerogenes, Escherichia coli, Proteus sp.* or the like. The organisms believed to be most readily treated by an antibacterially effected amount of a compound of this invention is *Staphylococcus aureus.* The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of the formula (II) as hereinbefore defined or an ester thereof which process comprises the hydrogenation of a compound of the formula (V)

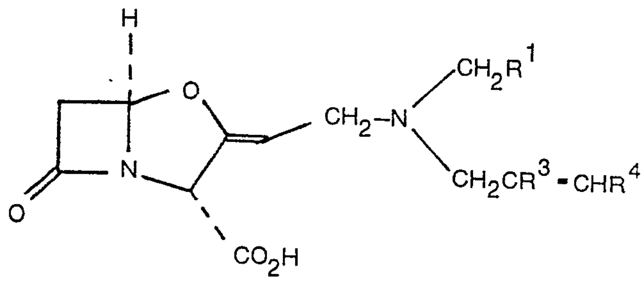

(V)

or ester thereof, wherein $R^1$ is isopropyl or isopropylene; and wherein $R^3$ is a hydrogen atom or lower alkyl group, $R^4$ is a hydrogen atom, a lower alkyl group, or a phenyl group optionally substituted with an inert organic group such as lower alkyl or lower alkoxy; or wherein $R^3$ and $R^4$ together represent a butadiene moiety; and thereafter if desired esterifying the zwitterion of the formula (IIa) as hereinbefore defined which was produced by the hydrogenation of the compound of the formula (V) or a hydrogenolysable ester thereof.

When used herein the term "hydrogenolysable ester" means an ester which on hydrogenation is cleaved to yield the parent carboxylic acid.

When used herein the term "lower-alkyl" means an alkyl group with 1—4 carbon atoms. Thus examples of suitable alkyl groups are methyl, ethyl, n-propyl, isopropyl, n-butyl, sec-butyl and iso-butyl.

Particularly suitable groups $CH_2CR^3{=}CHR^4$ for use in the compounds of formula (V) include the following,

$CH_2CH{=}CH_2$, $CH_2CH{=}CHC_6H_5$, $CH_2C(CH_3){=}CH_2$,

$CH_2C(C_2H_5){=}CH_2$, $CH_2C(nC_3H_7){=}CH_2$, $CH_2C(CH_3){=}CHCH_3$, $CH_2C(CH_3){=}C(CH_3)_2$,

$CH_2C(CH_3){=}CHC_2H_5$, $CH_2C(CH_3){=}CHC_6H_5$ and $CH_2C_6H_5$.

Favoured groups $CH_2CR^3{=}CHR^4$ are

$CH_2CH{=}CHCH_3$, $CH_2CH{=}CHC_6H_5$, $CH_2C(CH_3){=}CH_2$, $CH_2C(CH_3){=}CHC_6H_5$ and $CH_2C_6H_5$.

Particularly preferred groups $CH_2CR^3{=}CHR^4$ are

$CH_2C(CH_3){=}CH_2$, $CH_2C(CH_3){=}CHC_6H_5$ and $CH_2C_6H_5$.

A process according to this invention comprises the preceding hydrogenation when $CH_2CR^3CHR^4$ is a $CH_2C_6H_5$ group.

The hydrogenation is normally carried out in the presence of a transition metal catalyst.

The catalyst we have preferred to use is palladium, for example in the form of palladium on carbon (charcoal), palladium on barium sulphate, palladium on calcium carbonate, palladium black or the like.

A favoured catalyst is palladium on carbon (sometimes referred to as palladium on charcoal); for example 5%, 10%, 20% or 30% palladium on carbon. The high palladium content catalysts are particularly apt as smaller total weights of catalyst can be employed thereby avoiding possible problems associated with adsorption of product onto the carbon.

A low, medium or high pressure of hydrogen may be used in this reaction, for example from 1 to 6 atmospheres. In general if the catalyst used contains a lower percentage of palladium (for example 5% or 10% palladium) then better yields of the desired product are obtained using a pressure of about 3 to 5 atmospheres of hydrogen, for example about 4 atmospheres of hydrogen. In general if the catalyst used contains a higher percentage of palladium (for example 20% or 30% palladium) then acceptable yields of the desired product may also be obtained at low and medium pressures of hydrogen, for example about 1 to 2 atmospheres of hydrogen. We have found it convenient to use an atmospheric or slightly superatmospheric pressure of hydrogen in conjunction with higher palladium content catalysts.

The reaction is normally carried out at a non-extreme temperature, for example from 0°C to 30°C and more usually from 12°C to 25°C. It is generally convenient to carry out the reaction at ambient temperature.

Suitable solvents for carrying out the hydrogenation include ethanol, n-propanol, isopropanol, tetrahydrofuran, dioxane, ethyl acetate or mixtures of such solvents or such solvents in the presence of water. A favoured solvent is aqueous tetrahydrofuran. Further favoured solvents are ethanol or a mixture of isopropanol, tetrahydrofuran and water.

We have preferred to carry out the hydrogenation reaction on a hydrogenolysable ester of a compound of the formula (V) so that a compound of the formula (II) per se is formed by the hydrogenation. Such hydrogenation reaction proceeds at least in part via the formation of a compound

of the formula (V). Favoured hydrogenolysable esters include benzyl and substituted benzyl esters such as methoxybenzyl, nitrobenzyl (for example the p-nitrobenzyl ester), chlorobenzyl, bromobenzyl and like esters. A particularly suitable hydrogenolysable ester is the benzyl ester. A further particularly suitable hydrogenolysable ester is the p-methoxybenzyl ester.

Further favoured hydrogenolysable ester groups include those groups $CH_2CR^3{=}CHR^4$ that have been specified hereinbefore as being favoured for removal from a nitrogen atom by hydrogenolysis.

If the hydrogenation is performed on non-hydrogenolysable esters of the compound of the formula (V) then naturally an ester of the compound of the formula (II) results.

The product may generally be isolated from the reaction mixture by filtering off the solids (the catalyst, which should be well washed to remove the product) and then evaporating the solvent, preferably under low pressure, to yield the initial product. Further purification may be effected by such conventional methods as chromatography over cellulose or other mild stationary phase eluting with a $C_{1-4}$ alkanol optionally in the presence of water and optionally in the presence of tetrahydrofuran. Evaporation of the combined active fraction (identified by aqueous potassium permanganate spray on tlc) then yields the desired compound in pure form. The desired product is normally obtained in crystalline form (unless it is an unsalted ester). Trituration under ethanol, isopropanol or the like $C_{1-4}$ alkanol or other conventional solvent such as a ketone, ether or ester solvent or other conventional solvent (for example of up to 6 carbon atoms and more suitably of up to 4 carbon atoms) may also be used to aid crystallisation. Recrystallisation from ethanol or the like may also be employed. The solvent used in such processes may advantageously be moist. The preceding work up procedures successfully separate the desired compound from other products of the hydrogenation such as the disecondary-butylamine derivative.

Zwitterionic compounds, such as those of the formula (II) may be obtained from high yielding reactions by the addition of $C_{1-4}$ alkanol such as cold ethanol or the like to the initial product.

The initial product of the lower yielding reactions may contain considerable impurities so that it may be advantageous to wash the initial product by dissolving in a water immiscible organic solvent and extracting into water. Evaporation of the aqueous phase, preferably under a good vacuum, then yields a purer product which may be further purified if desired as previously described.

Unsalted esters of the compounds of the formula (II) tend to be oils so that it is often more convenient for handling to convert them into solid acid addition salts, for example by reaction with one equivalent of an acid. Alternatively the non-hydrogenolysable ester of the compound of the formula (V) may be hydrogenated in the presence of one equivalent of an acid, that is they may be hydrogenated in the form of their acid addition salt.

The compounds of the formula (II) may be hydrogenated in the form of their acid addition salts with a strong acid but this is not a preferred form of the process of this invention.

The present invention also provides a process for the preparation of an ester of a compound of the formula (II) which process comprises the reaction of the compound of the formula (II) with an esterifying agent.

The zwitterionic compound of the formula (II) may be dissolved or suspended in a solvent such as dimethylformamide, hexamethylphosphoramide, dichloromethane, ethyl acetate or other non-esterifiable solvents and therein esterified. Suitable temperatures for such a reaction range from about 0° to about 25°C. Suitable esterifying reagents include reactive halides and their equivalents, alkyl oxonium salts and the like.

When a reagent such as a reactive iodide, chloride, bromide, tosylate, mesylate or the equivalent is used, the resulting salt is generally suitable for use in a composition of this invention. Alternatively, the salt may be converted to a free base or alternative salt. When an alkyl oxonium salt is used, it is preferred to convert the resulting tetrafluoroborate to the free base or alternative salt. The various afore-mentioned salts may be converted to the free base by neutralisation, for example by contacting a solution of the salt in water with an organic phase, neutralising the salt by adding a base and extracting the liberated amine into the organic phase. This amine may thereafter be re-salted by reacting with an appropriate acid, for example in a dry organic solvent. It is generally preferred to use not more than one equivalent of acid for this process. Alternatively, the originally formed salt may be converted into the alternative salt using an ion exchange material for example, by passing an aqueous solution of one salt through a bed of an anion exchange resin in the form of the form of the desired salt such as the chloride form.

The salts may normally by obtained in solid form by dissolving in a fairly polar organic solvent (such as ethanol, tetrahydrofuran or the like) and then precipitating using a non-polar solvent such as diethyl ether, cyclohexane or the like.

The salts of the esters of the compounds of the formula (II) may normally be obtained in crystalline form by conventional methods such as trituration under (or crystallisation or recrystallisation from) a suitable organic solvent such as ether, acetone, acetonitrile, tetrahydrofuran or the like.

The present invention also provides a process for the preparation of an ester of the compound of the formula (II) which process comprises the reaction of an acid addition salt of the compound of the formula (II) with an alcohol in the presence of a condensation promoting agent.

Suitable condensation promoting agents for use in this process include carbodiimides such as

dicyclohexylcarboxiimide and the chemical equivalents thereof.

The acid addition salt may be formed in situ or may be preformed. The acid employed will normally be a strong acid such as a methane sulphonic acid, p-toluene sulphonic or the like or trifluoroacetic acid or the like.

The reaction is normally carried out in an inert organic solvent. When the ester being formed is that of a liquid alcohol it is convenient to use that alcohol as the solvent or as part of the solvent system. The esterification is generally performed at a non-extreme temperature such as 0° to 35°C, for example from about 10° to 25°C. Conveniently the reaction may be performed at ambient temperature.

Since the compound of the formula (V) and its salts and esters are of use as intermediates they form part of this invention. Suitably the compounds of the formula (V) are in the form of an ester of a type hereinbefore described. Suitably the compounds of the formula (V) are in the form of a salt such as in alkali metal salt, for example the lithium salt.

The intermediates of the formula (V) and its salts and esters may be prepared by the methods of British Application No. 41887/75, U.S. Serial No. 731928, Belgian Patent No. 847044 or West German Offenlegungsschrift P2646003.7.

### Example 1

*Benzyl 9-N,N-bis(2'-methylallyl)aminodeoxyclavulanate*

Benzyl dichloroacetylclavulanate (5 g; 12.5 mm) in dry dimethylformamide (75 $cm^3$) at 0° was treated with bis(2-methylallyl)amine (1.9 equivalents), and stirred at 0° for 2 hours. The mixture was poured into ethylacetate (250 $cm^3$) and washed with water (5 × 100 $cm^3$) and saturated brine (5 × 100 $cm^3$), dried (anhydrous magnesium sulphate) and evaporated to an oil. This oil was chromatographed on silica eluting with ethylacetatecyclohexane (1:1). Fractions were collected containing the title compound Rf ($SiO_2$/ethylacetate-cyclohexane; 1:1) = 0.82 (detection by aqueous potassium permanganate spray). Combined fractions were evaporated to yield an oil, 1.46 g (29%).

$\nu$ (film) 1805, 1750, 1695, 895, 755, 700 $cm^{-1}$. $\delta$ ($CDCl_3$) 1.67 (6H, s), 2.78 (4H, s), 2.95 (1H, d, *J* 17Hz), 3.03 (2H, d, *J* 7Hz), 3.42 (1H, dd, *J* 17 and 3Hz), 4.67 (1H, t, *J* 7Hz), 4.80 (4H, broad s), 5.03 (1H, s), 5.17 (2H, s), 5.61 (1H, d, *J* 3Hz), 7.33 (5H, s).

### Example 2

*9-N-Isobutylaminodeoxyclavulanic acid*

Benzyl 9-N,N-bis(2'-methylallyl)aminodeoxy clavulanate (0.8 g; 2.02 mm) in ethanol (30 $cm^3$) was hydrogenated in the presence of palladium on charcoal 10% (0.3 g) for 10 minutes at atmospheric pressure; the catalyst had been pre-hydrogenated for 20 minutes. The catalyst was filtered off and washed with aqueous ethanol (50 $cm^3$), the filtrate was evaporated in vacuo and ethanol added, after cooling at 0° a crystalline solid was filtered off and washed with cold ethanol, drying in vacuo afforded the title compound as a white crystalline solid; yield=190 mg (37%) Rf ($SiO_2$/butanol-propan-2-ol-water; 7:7:6)=0.45. $\nu$ (Nujol) 1805, 1690, 1610, 1570, 1185, 1110, 1080, 1070, 1045, 1020, 1005, 930, 895, 885, 857, 810, 758 $cm^{-1}$. $\nu$ (KBr) 3570 (broad), 3350 (broad), 3220 (broad), 2840—2740, 2450 (broad), 1780, 1695, 1600 (very broad), 1470, 1392, 1320, 1295, 1110, 1045, 1020, 1003, 987, 932, 893, 885, 812, 750 $cm^{-1}$. $\delta$ ($D_2O$) 0.94 (6H, d, *J*, 6Hz), 1.90 (1H, m), 2.82 (2H, d, *J* 6Hz), 3.07 (1H, d, *J* 17Hz), 3.57 (1H, dd, *J* 17 and 3Hz), 3.70 (2H, d, *J* 7Hz), 4.77 (1H, 6, *J* 7Hz), 4.96 (1H, s), 5.73 (1H, d, *J* 3Hz).

### Example 3

*Benzyl 9-*N*-Isobutyl-*N*-(2'-methyl-3'-phenylallyl)aminodeoxyclavulanate*

Benzyl dichloroacetylclavulanate (7.16 g; 17.9 mM) in dry dimethylformamide (85 $cm^3$) at −12° was treated with 1.9 equivalents of *N*-isobutyl-*N*-(2-methyl-3-phenylallyl)amine and stirred at −15° for 15 minutes then for 45 minutes between −10 and 0°. The mixture was poured into iced ethylacetate (250 $cm^3$) and washed with water (5 × 100 $cm^3$), saturated brine (5 × 100 $cm^3$), dried (anhydrous magnesium sulphate) and evaporated in the presence of toluene to a small volume. This crude product was chromatographed on silica eluting with ethylacetate-cyclohexane; 1:2. Fractions were collected containing the title compound Rf ($SiO_2$/ethylacetate-cyclohexane; 1:2)=0.89. Combined fractions were evaporated to afford the title compound as an oil, yield=1.60 g (19%), $\nu$ (film) 1808, 1750, 1690, 745, 700 $cm^{-1}$. $\delta$ ($CDCl_3$) 1.85 (6H, d, *J* 6Hz), 1.50—2.20 (6H, broad m), 2.92 (1H, d, *J* 17Hz), 2.94 (2H, s), 3.39 (1H, dd, *J* 17 and 3 Hz), 4.73 (1H, broad t, *J* 7Hz), 5.07 (1H, broad s), 5.17 (2H, s), 5.60 (1H, d, *J* 3Hz), 6.35 (1H, broad s), 7.25 and 7.32 (10 H, 2 x s).

### Example 4

*9-*N*-Isobutylaminodeoxyclavulanic acid*

Benzyl 9-*N*-(2'-methyl-3'-phenylallyl)-*N*-isobutylaminodeoxyclavulanate (1.44 g; 3.04 mM) in ethanol (25 $cm^3$) was hydrogenolysed in the presence of palladium on carbon (10% Pd), 0.5 g (which had been prehydrogenated for 15 minutes), for 45 minutes at atmospheric pressure. The catalyst was filtered off and washed with ethanol (50 $cm^3$), then with aqueous ethanol (100 $cm^3$), the aqueous

washing was collected separately and was evaporated to yield the title compound as a white crystalline solid. The solid was washed with a little cold ethanol, drying afforded 248 mg of the title compound. The ethanolic catalyst washings and the solvent from the hydrogenolysis were evaporated, ethanol added (10 $cm^3$) and cooled, crystals were filtered off and dried to yield a further 17 mg of the title compound, total yield=265 mg (34%). The infrared and proton magnetic resonance spectra were identical to the product obtained by hydrogenolysis of benzyl 9-*N,N*-bis(2′-methylallyl)amino-deoxyclavulanate.

Example 5

*Benzyl 9-*N*-(2′-methylallyl)-*N*-(3″-methylallyl)aminodeoxyclavulanate*

Benzyl dichloroacetylclavulanate (7.4 g; 18.5 mM) in dry dimethylformamide (50 $cm^3$) at 0° was treated with *N*-(2′-methylallyl)-*N*-(3″-methylallyl)amine (1.9 equivalents) and stirred at 0° for 45 minutes. The mixture was poured into ethyl acetate (200 $cm^3$) and washed with water (6×100 $cm^3$) and saturated brine (6×100 $cm^3$), dried (anhydrous magnesium sulphate) and evaporated to an oil. This oil was chromatographed on silica eluting with ethyl acetate-cyclohexane (1:2), fractions were collected Rf ($SiO_2$/ethyl acetate-cyclohexane; 1:2)=0.5 (detection by aqueous potassium permanganate spray). Combined fractions were evaporated to yield the title compound as an oil, yield=2.7 g (37%). $\nu$ (film) 1805, 1750, 1700, 1450, 1380, 1305, 1230, 1175, 1120, 1080, 1042, 1015, 970, 895, 740, 700 $cm^{-1}$. $\delta$ ($CDCl_3$) 1.58—1.80 (6H, m) 2.81 (4H, broad s), 2.95 (1H, d, *J* 17 Hz), 3.08 (2H, d, *J* 7 Hz), 3.42 (1H, dd, *J* 17 and 3 Hz), 4.68 (1H, t, *J* 7 Hz), 4.79 (2H, broad s), 5.03 (1H, s), 5.15 (2H, s), 5.36—5.56 (2H, broad m), 5.60 (1H, d, *J* 3 Hz), 7.32 (5H, s).

Example 6

*9-*N*-Isobutylaminodeoxyclavulanic acid*

Benzyl 9-*N*-(2′-methylallyl)-*N*-(3″-methylallyl) aminodeoxyclavulanate (1.37g) in ethanol (30 $cm^3$) was hydrogenolysed at atmospheric pressure in the presence of 10% palladium on charcoal (0.5 g, which had been prehydrogenated for 20 minutes) for 25 minutes. The catalyst was filtered off and washed with aqueous ethanol, the filtrate was evaporated and ethanol added. The resulting crystals were filtered off cold and dried. This product was chromatographed on cellulose eluting with butanol-isopropanol-water; 4:4:1. Fractions were collected containing the title compound in low yield Rf ($SiO_2$/butanol-isopropanol water; 7.76) = 0.46 (detection by aqueous potassium permanganate spray).

Example 7

*Benzyl 9-*N*-(2′-methylallyl)-*N*-(3″-transphenylallyl) aminodeoxyclavulanate*

Benzyl dichloroacetylclavulanate (5.9 g; 14.8 mM) in dimethylformamide (40 $cm^3$) at 0° was treated with *N*-(2-methylallyl)-*N*-(3′-*trans*phenylallyl) amine (1.9 equivalents) and stirred at 0° for $1\frac{3}{4}$ hours. The mixture was then poured into ethyl acetate (250 $cm^3$) and washed with water (5×100 $cm^3$) and saturated brine (5×100 $cm^3$), dried (anhydrous magnesium sulphate) and evaporated to an oil. This oil was chromatographed on silica eluting with ethylacetate-cyclohexane (1:2), fractions were collected containing the title compound; Rf ($SiO_2$/ethylacetate:cyclohexane, 1:1)=0.76 (detection by aqueous potassium permanganate spray). Combined fractions were evaporated to yield an oil, 3.49 g (51%).

$\nu$ (film) 1805, 1750, 1700, 1495, 1450, 1305, 1230, 1175, 1120, 1080, 1045, 1015, 967, 895, 745, 695 $cm^{-1}$.

$\delta$ ($CDCl_3$) 1.71 (3H, s), 2.89 (2H, s), 2.93 (1H, d, *J*, 17Hz), 3.07 (2H, d, *J* 6Hz), 3.16 (2H, d, *J* 7Hz) 3.39 (1H, dd, *J* 17 and 3Hz), 4.72 (1H, t, *J* 7Hz), 4.75—4.95 (2H, broad m), 5.06 (1H, s), 5.16 (2H, s), 5.58 (1H, d, *J* 3Hz), 6.15 (1H, dt, *J*16 and 6Hz), 6.45 (1H, d, *J* 16Hz), 7.15—7.50 (10H, m).

Example 8

*9-*N*-Isobutylaminodeoxyclavulanic acid*

Benzyl 9-*N*-(2′-methylallyl)-*N*-(3″-*trans* phenyl allyl) aminodeoxyclavulanate (1.1 g) in ethanol (40 $cm^3$) was hydrogenolysed for 20 minutes at atmospheric pressure in the presence of 10% Palladium on charcoal (300 mg; which had been prehydrogenated for 10 minutes). The catalyst was filtered off and washed with aqueous ethanol. The filtrate was evaporated and ethanol added, the resulting crystals were filtered off cold and dried. This product was chromatographed on cellulose eluting with butanol-isopropanol-water; 4:4:1. Fractions were collected containing the title compound in low yield, Rf ($SiO_2$/butanol-isopropanol-water; 7:7:6)=0.46 (detection by aqueous potassium permanganate spray).

Example 9

*Benzyl 9-*N*-(2′-methylallyl)-*N*-(2″-methyl-3″-phenylallyl)amino-deoxyclavulanate*

Benzyl 9-O-dichloroacetylclavulanate (6.30 g; 15.8 15.8 mmol) in acetonitrile (60 ml) at 4°C was treated with dropwise addition of N-(2′-methylallyl)-N-(2″-methyl-3″-phenylallyl)-amine (6 g; 30 mmol) in acetonitrile (60 ml). The reaction mixture was stirred at 4 to 10°C for 2 hours.

The acetonitrile was then removed under a reduced pressure and the resulting oil was dissolved in ethyl acetate. The solution was then washed with water, brine, dried ($MgSO_4$) and evaporated *in vacuo*.

Silica-gel column chromatography afforded the title compound as an oil in a 16% yield.

$\nu_{max}$ ($CHCl_3$): 1802, 1745, 1695 and 1600 (br) $cm^{-1}$. ($CDCl_3$): 1.70 (3H, s), 1.82 (3H, s), 2.84 (2H, s), 2.92 (2H, s), 2.90 (1H, d, *J* 17 Hz), 3.12 (2H, d, *J* 7 Hz), 3.38 (1H, dd, *J* 17 and 3 Hz), 4.70 (1H, br t, *J* 7 Hz), 4.82 (2H, br s), 5.06 (1H, s), 5.16 (2H, s), 5.60 (1H, d, *J* 3Hz), 6.36 (1H, s), 7.22 and 7.30 (10H, 2 x s).

Example 10

*9-*N*-Isobutylaminodeoxyclavulanic acid*

Benzyl-9-N-(2-methylallyl)-N-(2″-methyl-3″-phenylallyl) aminodeoxyclavulanate (1.0 g; 2 mmol) in ethanol (20 ml) was carefully added to a pre-hydrogenated mixture of 10% palladium on charcoal (300 mg) in ethanol (30 ml). The mixture was then hydrogenated at atmospheric pressure for 30 minutes. The catalyst was then filtered off and washed well with aqueous ethanol. The filtrate plus washings were then evaporated to dryness under a reduced pressure. Cellulose column chromatography afforded the title compound as a white crystalline solid in 30% yield.

Example 11

*Benzyl 9-*N*-(iso-butyl)-*N*-(2′-methylallyl)aminodeoxyclavulanate*

*N*-Isobutyl-*N*-(2-methylallyl)amine (7.25 g, 57 mmol) in dimethylformamide (30 ml) was added dropwise to a solution of benzyl dichloroacetylclavulanate (12.0 g, 30 mmol) in dimethylformamide (200 ml) at −10°C. After 2 hours at this temperature the reaction mixture was poured into water and extracted with ethyl acetate. The organic phase was washed several times with brine, dried ($MgSO_4$) and evaporated to a yellow oil. Chromatography on silica gel (elution: petrol/ethylacetate: 3/1 grading to 2/1) afforded the title ester as an oil, 2.98 g (25%) I.R. ($CHCl_3$) 1802, 1745, 1700, and 895 $cm^{-1}$. N.M.R. ($CDCl_3$) 0.84 (6H, d, *J* 7 Hz), 1.52—1.88 (1H, m) 1.68 (3H, s), 2.00 (2H, d, *J* Hz), 2.79 (2H, s), 2.94 (1H, d, *J* 17 Hz), 3.06 (2H, d, *J* 8 Hz), 3.41 (1H, dd, *J* 17 and 3 Hz), 4.68 (1H, bt, *J* 8 Hz), 4.78 (2H, bs), 5.03 (1H, bs), 5.17 (2H, s), 5.61 (1H, d, *J* 3 Hz), and 7.32 (5H, s).

Example 12

*9-*N*-Isobutylaminodeoxyclavulanic acid*

10% Pd-C (0.83 g) in ethanol (80 ml) was hydrogenated for 15 minutes at 1 atmosphere of hydrogen. Benzyl 9-*N*-(isobutyl)-*N*-(2′-methylallyl)aminodeoxyclavulanate (2.5 g, 6.28 mmol) in ethanol (30 ml) was added and the hydrogenation continued for 45 minutes. The catalyst was then filtered off through celite and the pad was washed with some ethanol. These combined washings were evaporated to a yellow oil which crystallised from ethanol (0.120 g).

The filter pad, above, was now washed with 50% aqueous ethanol (150 ml). Evaporation afforded the title material as a white solid (0.70 g).

Combination of all the mother liquors and evaporation afforded a dark oil which was chromatographed on silica gel (elution: ethylacetate/*iso*-propanol/water: 5/2/1 grading to 5/4/3) to provide more of the title product (0.115 g).

Total yield of required product=0.935 g (59% yield).

N.M.R., I.R., and t.l.c. characteristics were identical to an authentic sample.

Example 13

*Benzyl 9-*N*-Isobutyl-*N*-benzylaminodeoxyclavulanate*

Benzyldichloroacetylclavulanate (9.04 g; 23 mM) in dry dimethylformamide (50 $cm^3$) at − 10°C was treated with 1.9 equivalents of *N*-isobutyl-*N*-benzylamine (7 g; in 25 $cm^3$ dimethylformamide) over 15 mins and allowed to warm up to −3°. The reaction mixture was stirred for a further 75 minutes at 0°.

The reaction mixture was poured into ethyl acetate (300 $cm^3$) and washed with water (4×200 $cm^3$) and saturated brine (5×200 $cm^3$), dried (anhydrous magnesium sulphate), evaporated in the presence of toluene to small volume and chromatographed on silica eluting with ethyl acetate-cyclohexane; 1:3. Fractions were collected containing the title compound, Rf ($SiO_2$/ethylacetate-cyclohexane; 1:3)=0.54 (detection by aqueous potassium permanganate spray). Combined fractions were evaporated to yield an oil, 1.45 g (14.5%), $\nu$ (film) 1805, 1750, 1695, 1305, 1175, 1015, 740, 700 $cm^{-1}$, $\delta$ ($CDCl_3$) 0.84 (6H, d, *J* 6Hz), 1.5—2.0 (1H, broad m), 2.09 (2H, d, *J* 6Hz), 2.93 (1H, d, *J* 17 Hz), 3.11 (2H, d, *J* 7 Hz), 3.40 (1H, dd, *J* 17 Hz and 3 Hz), 3.43 (2H, s), 4.72 (1H, dt, *J* 7 and 1 Hz), 5.04 (1H, d, *J* 1 Hz), 5.16 (2H, s), 5.59 (1H, d, *J* 3 Hz), 7.25 and 7.32 (10H, 2 x s).

Example 14

*9-*N*-isobutylaminodeoxyclavulanic acid*

Benzyl 9-*N*-isobutyl-*N*-benzylaminodeoxyclavulanate (1.31 g; 3.02 mM) in ethanol (25 $cm^3$) and water (3 $cm^3$) was hydrogenolysed at atmospheric pressure in the presence of 10% palladium on charcoal (430 mg; 33% w/w) for 3 hours. The catalyst was filtered off and washed with aqueous ethanol (150 $cm^3$). The filtrate was evaporated and ethanol added resulting in the formation of white crystals. These were filtered off cold (0°) and washed with cold ethanol, drying afforded the title compound as a white crystalline solid, yield=0.33 g (43%) Rf ($SiO_2$/ethyl acetate/ethanol/water;

5:2:2)=0.36. $\nu$ (Nujol) 1805, 1692, 1610, 1565, 1305, 1187, 1110, 1045, 1038, 1020, 1005, 932, 895, 880, 858, 812, 758 $cm^{-1}$. The proton magnetic resonance spectrum was consistent with the required product.

## Example 15

*Phenacyl 9-N-bis (2'-methylallyl)aminodeoxyclavulanate*

Phenacyl 9-dichloroacetyl clavulanate (5.0 g; 11.7 mmol) in dry dimethylformamide (50 ml) was cooled to 0°C and bis (2'-methylallyl) amine (2.8 g; 22.2 mmol) in dry dimethylformamide (30 ml) was added dropwise. After stirring at 0°C for 1½ hours the mixture was poured into ethyl acetate and washed several times with water and then dried ($MgSO_4$). After filtration the ethyl acetate was removed *in vacuo* to give a yellow oil, which on column chromatography afforded the required compound as a yellow gum, yield=1.57 g (32%).

$[\alpha]_D^{20}$+11.0° (c. 1%, $CHCl_3$). Found: C, 67.80; H, 6.70; N, 6.38%, $C_{24}H_{28}N_2O_5$ requires: C, 67.91; H, 6.65; N, 6.60% $\nu_{max}$ ($CHCl_3$) 1805, 1760 and 1605 $cm^{-1}$; $\delta$ ($CDCl_3$) 1.72 (6H, s), 2.7—3.22 (7H, m), 3.45 (1H, dd, $J$ 17 and 3 Hz), 4.71—5.0 (5H, m), 5.19 (1H, s), 5.37 (2H, s), 5.66 (1H, d, $J$ 3 Hz), 7.32—8.00 (5H, m).

## Example 16

*Allyl 9-N,N-bis(2'-methylallyl)aminodeoxyclavulanate*

Allyl dichloroacetylclavulanate (5 g; 14.3 mM) in dry dimethylformamide (50 $cm^3$) at 0° was treated with bis (2-methylallyl)amine (1.9 equivalents) and stirred 2½ hours at between 0° and 5°C. The mixture was poured into ethylacetate (250 $cm^3$) and washed with water (5×200 $cm^3$) and saturated brine (5×150 $cm^3$), dried (anhydrous magnesium sulphate) and evaporated to an oil. This oil was chromatographed on silica eluting with ethylacetate-cyclohexane; 1:1. Fractions were collected containing the title compound, Rf ($SiO_2$/ethylacetate-cyclohexane; 1:1)=0.90 (detection by aqueous potassium permanganate spray). Combined fractions were evaporated *in vacuo* to yield an oil, 1.33 g (27%), $\nu$ (film) 1808, 1750, 1695, 1450, 1370, 1308, 1232, 1180, 1120, 1015, 940, 895 $cm^{-1}$. $\delta$ ($CDCl_3$) 1.71 (6H, s), 2.83 (4H, s), 2.98 (1H, d, $J$ 17 Hz), 3.07 (2H, d, $J$, 7 Hz), 3.45 (1H, dd, $J$ 17 and 3 Hz), 4.55—4.94 (7H, m), 5.02 (1H, s), 5.17—5.5 (2H, m), 5.63 (1H, d, $J$ 3 Hz), 5.6—6.16 (1H, m).

## Example 17

*(2-Methylallyl) 9-N-(Isobutyl)-N-(2''-Methylallyl) aminodeoxyclavulanate*

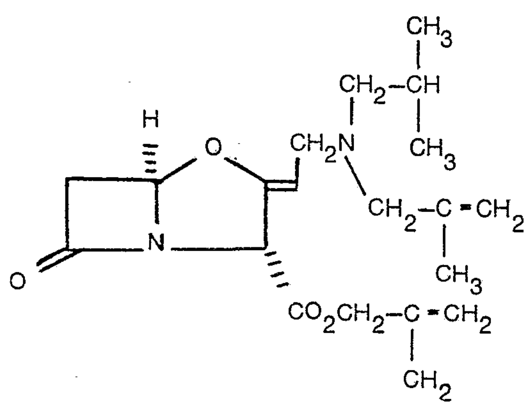

(2-Methylallyl)-9'-O-dichloroacetylclavulanate (9.05 g; 24.8 mmol) in anhydrous dimethylformamide (90 ml) was cooled with stirring to −20°C. N-(Isobutyl)-N-(2-methylallyl) amine (6.0 g; 47 mmol) in anhydrous dimethylformamide (60 ml) was added dropwise over a period of 20 minutes. The reaction mixture was allowed to warm to −10°C and stirring continued for 2 hours.

The solution was poured into a cold mixture of ethyl acetate and water and shaken. The aqueous layer was extracted with more ethyl acetate. The combined organic layers were washed with water, dried ($MgSO_4$) and evaporated to an oil. Column chromatography afforded the title compound as a colourless oil in 23% yield.

$\nu_{max}$ ($CHCl_3$): 1805, 1750 and 1700 $cm^{-1}$. $\delta$ ($CDCl_3$): 0.85 (6H, d $J$ 7Hz), 1.70 (3H, s), 1.77 (3H, s), 1.57 to 1.88 (1H, m), 2.05 (2H, d, $J$ 7Hz), 2.84 (2H, s), 2.94 (1H, d, $J$ 17Hz), 3.11 (2H, d, $J$ 7Hz), 3.46 (1H, dd, $J$ 17 and 3Hz), 4.58 (2H, s), 4.74 (1H, broad t, $J$ 7Hz), 4.81 (2H, broad s), 4.99 (2H, broad s), 5.06 (1H, s), and 5.66 (1H, d, $J$ 3Hz).

Example 18

*9-N-Isobutylaminodeoxyclavulanate*

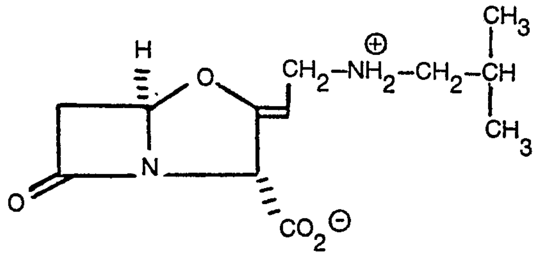

(2-Methylallyl)-9′-N-(isobutyl)-N-(2″-methylallyl) aminodeoxyclavulanate (1.5 g; 4 mmol) in ethanol (20 ml) was carefully added to a pre-hydrogenated mixture of 10% palladium on charcoal (0.5 g) in ethanol (20 ml). The mixture was hydrogenated at 1 atmosphere until the uptake of hydrogen ceased.

The mixture was filtered through a celite pad and the "cake" washed with aqueous ethanol. The filtrate plus washings were evavorated to dryness resulting in a yellow solid which an addition of a small amount of ethanol gave a white solid. Filtration afforded the title compound in 20% yield. Spectral data was consistent with an authentic example.

Example 19

*Composition*

a) A solution for injection may be prepared by dissolving 100 mg of sterile 9-*N*-isobutylaminodeoxyclavulanic acid in 1 ml of sterile water.

b) A solution for injection may be prepared by dissolving 50 mg of sterile 9-*N*-isobutylaminodeoxyclavulanic acid and 250 mg of sterile sodium amoxycillin in 1 ml of sterile water.

c) A solution for injection may be prepared by dissolving 125 mg of sterile 9-N-isobutylaminodeoxyclavulanic acid and 125 mg of sterile cephaloridine in 1.5 ml of sterile water.

Description 1

*Allyl 9-O-dichloroacetylclavulanate*

Allylclavulanate (6.3 g; 26 mm) in dichloromethane (100 $cm^3$) at —30°C was treated with pyridine (3 $cm^3$) and dichloroacetylchloride (1 equivalent) and stirred for 10 mins at —20°C. The mixture was diluted with dichloromethane (100 $cm^3$), and washed with aqueous citric acid solution (10%; 50 $cm^3$), water (3 × 100 $cm^3$), saturated brine (3 × 150 $cm^3$), dried (anhydrous magnesium sulphate) and evaporated to an oil, yield = 8.3 g (90%), Rf ($SiO_2$/ethylacetate — cyclohexane 1:1) = 0.78. $\upsilon_{max}$ (film) 1808, 1755, 1695 $cm^{-1}$.

Description 2

*Phenacyl-9-O-Dichloroacetylclavulanate*

Phenacyl clavulanate (14.26 g; 45 mmol) in dry methylene chloride (100 ml) was treated with pyridine (4 mls, 50.6 mmole) at room temperature. The reaction mixture was cooled to —30°C and dichloroacetyl chloride (4.3 mls, 1.2 equivalents) in dry methylene chloride (15 ml) was added dropwise. After stirring at —30°C for 30 minutes the mixture was allowed to warm to 0°C and was then poured into dilute hydrochloric acid. The organic phase was separated and washed with more dilute hydrochloric acid, sodium bicarbonate solutions, water, brine and then dried ($MgSO_4$). After filtration the methylene chloride was removed *in vacuo* to afford a yellow oil which crystallised on trituration with ether. Yield=11.21 g, 58%. A sample was recrystallised from ether gave the following physical characteristics:

m.p. 75—77°C $[\alpha]_D^{20}$+16.1°, (c. 1%; $CHCl_3$).

$\upsilon_{max}$ (kBr): 1795, 1750 and 1590 $cm^{-1}$.

Found: C, 50.67; H, 3.30; N, 3.30; Cl, 16.43%.

$C_{18}H_{15}NO_7Cl_2$ requires C, 50.47; H, 3.51; N, 3.27; Cl, 16.59%.

Description 3

*(2-Methylallyl)clavulanate*

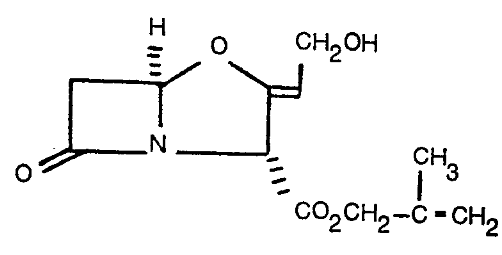

Sodium clavulanate (25 g; 83 mmol) in anhydrous dimethylformamide (100 ml) was treated at room temperature over a period of 15 minutes with methallylchloride (15.1 g; 166 mmol) in dimethylformamide (50 ml) plus a catalytic amount of sodium iodide. The mixture was then stirred at room temperature overnight.

The mixture was then poured into an ethyl acetate/water mixture and shaken. The two layers were separated and the aqueous layer extracted with ethyl acetate. The organic layers were combined, washed with water, washed with a saturated solution of sodium chloride, dried ($MgSO_4$) and evaporated to give the title compound as an oil in 73% yield.

$\nu_{max}$ ($CHCl_3$): 1810, 1750, 1695 and 1660 $cm^{-1}$. $\delta$ ($CDCl_3$): 1.75 (4H, s), 3.05 (1H, d, *J* 17Hz), 3.50 (1H, dd, *J* 17 and 3Hz), 4.23 (2H, d, *J* 7Hz), 4.58 (2H, s), 4.93 (1H, t, *J* 7Hz), 5.00 (2H, broad s), 5.09 (1H, m), and 5.70 (1H, d, *J* 3 Hz).

Description 4

*(2-Methylallyl)-9'-O-Dichloroacetylclavulanate*

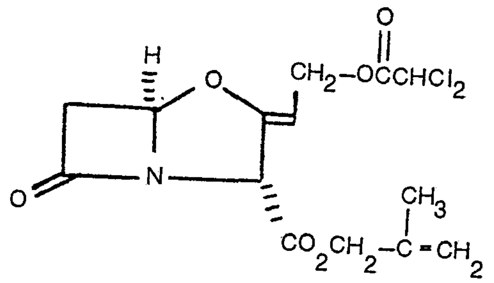

(2-Methylallyl)clavulanate (15.3 g; 60 mmole) in anhydrous dichloromethane (150 ml) was treated at room temperature with anhydrous pyridine (4.8 ml; 60 mmol) and was added dropwise over a period of 10 minutes. The reaction mixture was stirred at −40°C for 1 hour and then allowed to warm to 0°C.

The mixture was poured into cold dilute hydrochloric acid and separated. The organic layer was then washed several times with more dilute hydrochloric acid, water and a saturated solution of sodium chloride, dried ($MgSO_4$) and evaporated to an oil affording the title compound in 87% yield.

$\nu_{max}$ ($CHCl_3$): 1810, 1755 and 1700 $cm^{-1}$.

($CDCl_3$): 1.76 (3H, s), 3.10 (1H, d, *J* 17Hz), 3.56 (1H, dd, *J* 17 and 3Hz), 4.60 (2H, s), 4.78 to 5.00 (5H, m), 5.13 (1H, s), 5.77 (1H, d, *J* 3Hz), and 5.93 (1H, s).

Demonstration 1

9-N-Isobutylaminodeoxyclavulanic acid and 9-N-n-butylaminodeoxyclavulanic acid have been compared as synergists in vivo by the subcutaneous route against 2 strains of $R_{TEM}$ E. Coli in intraperitoneal mouse infections. On each occasion the isobutylamine was more active then the n-butylamine, producing a lower $CD_{50}$ for amoxycillin. These results are tabulated below.

*Comparative subcutaneous synergistic activity of 9-*N*-Isobutylaminodeoxyclavulanic acid and 9-*N*-n-butylaminodeoxyclavulanic acid with amoxycillin against mouse E. Coli infections*

| | $CD_{50}$ mg/kg × 2 | | | |
|---|---|---|---|---|
| | E. Coli E96 | | E. Coli E124 | |
| | P392 | P409 | P397 | P412 |
| Amoxycillin alone | > 1000 | > 1000 | > 1000 | > 1000 |
| Amoxycillin + 2 mg/kg 9-N-n-butylaminedeoxy-clavulanic acid | 17.5 | 7.2 | 17.5 | 11.5 |
| 9-N-isobutylaminodeoxy-clavulanic acid | 13.5 | 4.4 | 10.5 | 8 |

**Claims**

1. The compound of the formula (II):

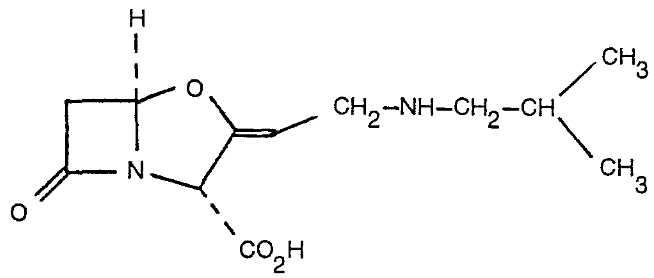

(II)

or an ester or acid addition salt of such an ester.

2. A compound as claimed in claim 1 when in pharmaceutically acceptable form.

3. 9-N-Isobutylaminodeoxyclavulanic acid.

4. 9-N-Isobutylaminodeoxyclavulanic acid when in crystalline form.

5. A compound of the formula (III) or (IV):

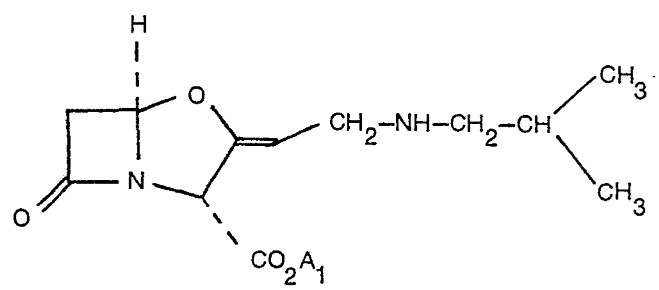

(III)

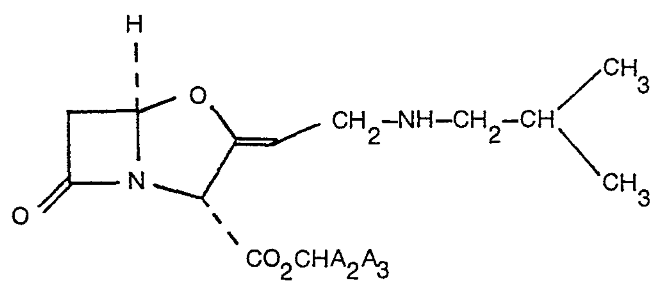

(IV)

wherein $A_1$ is an alkyl group of 1—6 carbon atoms optionally substituted by an alkoxyl or acyloxyl group of 1—7 carbon atoms; $A_2$ is an alkenyl or alkynyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

6. A compound of the formula (IV) as claimed in claim 5 wherein $A_2$ is phenyl, p-methoxyphenyl or p-nitrophenyl, and $A_3$ is hydrogen.

7. A compound as claimed in any of claims 2, 5 or 6 when in the form of a hydrochloride, phosphate, sulphate, methanesulphonate, toluenesulphonate, citrate, malate, acetate, lactate, tartrate, propionate or succinate acid addition salt.

8. A pharmaceutical composition which comprises a compound as claimed in any of claims 2 to 7 and a pharmaceutically acceptable carrier.

9. A pharmaceutical composition as claimed in claim 8 wherein the compound of formula (II) in crystalline form is adapted for administration by injection or infusion.

10. A pharmaceutical composition as claimed in claim 8 or claim 9 wherein an injectable aqueous solution contains not less than 15% w/w of the compound of the formula (II).

11. A composition as claimed in any of claims 8 to 10 which also contains a $\beta$-lactam antibiotic.

12. A composition as claimed in claim 11 which comprises benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, the acetoxymethyl, pivaolyloxymethyl, $\alpha$-ethoxycarbonyloxyethyl or phthalidyl ester of ampicillin, benzylpenicillin or amoxycillin, hetacillin, metampicillin, the phenyl or indanyl $\alpha$-esters of carbenicillin or ticarcillin, cefatrizine, cephaloridine, cephalothin, cefazolin, cephalexin, cephacetrile, cephamandole nafate, cephapirin, cepradine, 4-hydroxycephalexin, cafeparole or cephaloglycin.

13. A composition as claimed in any of claims 8 to 12 which comprises amoxycillin trihydrate or a pharmaceutically acceptable salt of amoxycillin.

14. A composition as claimed in any of claims 8 to 12 which comprises benzylpenicillin or a pharmaceutically acceptable salt of benzylpenicillin.

15. A composition as claimed in any of claims 8 to 12 which comprises ampicillin or a pharmaceutically acceptable salt of ampicillin.

16. A composition as claimed in any of claims 8 to 12 which comprises mezlocillin or a pharmaceutically acceptable salt of mezlocillin.

17. A composition as claimed in any of claims 8 to 12 which comprises azlocilin or a pharmaceutically acceptable salt of azlocillin.

18. A composition as claimed in any of claims 8 to 12 which comprises ticarcillin or a pharmaceutically acceptable salt of ticarcillin.

19. A composition as claimed in any of claims 8 to 12 which comprises phenoxymethylpenicillin or a pharmaceutically acceptable salt thereof.

20. A composition as claimed in any of claims 8 to 12 which comprises cephaloridine.

21. A composition as claimed in any of claims 8 to 12 which comprises cefazolin or a pharmaceutically acceptable salt of cefazolin.

22. A composition as claimed in any of claims 8 to 21 wherein the ratio of the compound of the formula (II) to the $\beta$-lactam antibiotic is 1:1 to 1:3.

23. A pharmaceutical composition which comprises from 150 to 1000 mg of amoxycillin as trihydrate or pharmaceutically acceptable salt and from 25 to 500 mg of 9-N-isobutylaminodeoxyclavulanic acid and a pharmaceutically acceptable carrier.

24. A pharmaceutical composition which comprises 200 to 2000 mg of ticarcillin or di-sodium ticarcillin and from 75 to 250 mg of 9-N-isobutylaminodeoxyclavulanic acid and a pharmaceutically acceptable carrier.

25. A composition as claimed in any of claims 8 to 24 for use in the treatment of bacterial infections.

26. 9-N-Isobutylaminodeoxyclavulanic acid for use as a $\beta$-lactamase inhibitor or antibacterial agent.

27. A process for the preparation of a compound as claimed in claim 1 which process comprises the catalytic hydrogenation of a compound of the formula (V):

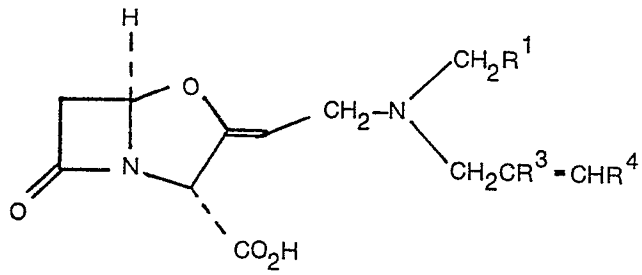

(V)

or ester thereof, wherein $R^1$ is isopropyl or isopropylene, and $R^3$ and $R^4$ together represent a butadiene moiety, and thereafter if necessary forming an acid addition salt of an ester.

28. A process as claimed in claim 27 wherein the compound of the formula (V) is in the form of a hydrogenolysable ester.

29. A process as claimed in claim 28 wherein the compound of the formula (V) is in the form of a benzyl ester.

## Revendications

1. Composée de formule (II):

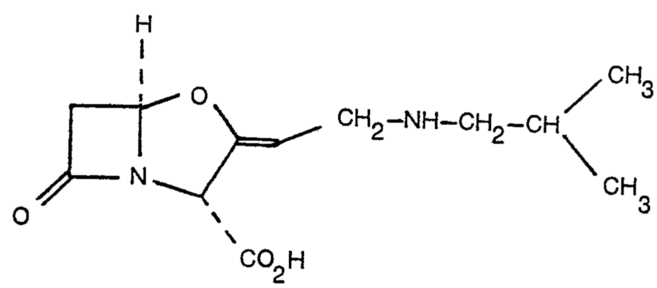

(II)

ou ester ou sel d'addition avec un acide d'un tel ester.

2. Composé suivant la revendication 1 sous une forme pharmaceutiquement acceptable.

3. Acide 9-N-isobutylaminodésoxyclavulanique.

4. Acide 9-N-isobutylaminodésoxyclavulanique sous une forme cristalline.

5. Composé de formule (III) ou (IV):

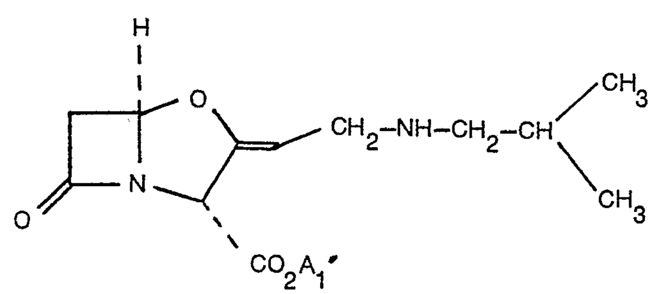

(III)

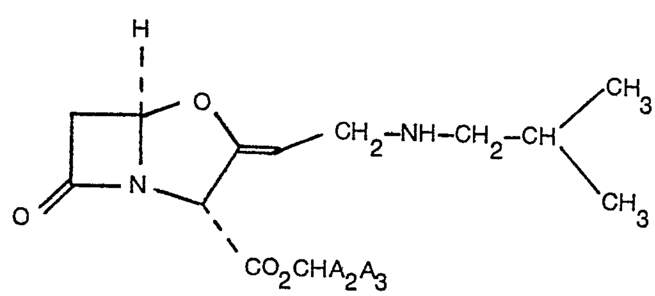

(IV)

dans lesquelles $A_1$ est un groupe alcoyle ayant de 1 à 6 atomes de carbone éventuellement substitué par un groupe alcoxy ou acyloxy ayant de 1 à 7 atomes de carbone; $A_2$ est un groupe alcényle ou alcynyle ayant jusqu'à 5 atomes de carbone ou est un groupe phényle éventuellement substitué par un atome de fluor, de chlore ou de brome ou par un groupe nitro ou alcoyle ou alcoxy ayant jusqu'à 4 atoms de carbone; et $A_3$ est un atome d'hydrogène, un groupe alcoyle ayant jusqu'à 4 atomes de carbone ou un groupe phényle éventuellement substitué par un atome de fluor, de chlore ou de brome ou par un groupe nitro ou alcoyle ou alcoxy ayant jusqu'à 4 atomes de carbone.

6. Composé de formule (IV) suivant la revendication 5, dans lequel $A_2$ est un groupe phényle, p-méthoxyphényle ou p-nitrophényle, et $A_3$ est de l'hydrogène.

7. Composé suivant l'une quelconque des revendications 2, 5 et 6 sous la forme d'un chlorhydrate, d'un phosphate, d'un sulfate, d'un méthanesulfonate, d'un toluène-sulfonate, d'un citrate, d'un malate, d'un acétate, d'un lactate, d'un tartrate, d'un propionate ou d'un succinate à titre de sel d'addition avec un acide.

8. Composition pharmaceutique renfermant un composé suivant l'une quelconque des revendications 2 à 7 et un excipient pharmaceutiquement acceptable.

9. Composition pharmaceutique suivant la revendication 8, dans laquelle le composé de formule (II) sous forme cristalline est adapté en vue d'une administration par injection ou infusion.

10. Composition pharmaceutique suivant la revendication 8 ou 9, dans laquelle une solution aqueuse injectable ne renferme pas moins de 15% p/p composé de formule (II).

11. Composition suivant l'une quelconque des revendications 8 à 10, renfermant également un antibiotique à cycle $\beta$-lactame.

12. Composition suivant la revendication 11, renfermant de la benzylpénicilline, de la phénoxyméthylpénicilline, de la carbénicilline, de l'azidocilline, de la propicilline, de l'ampicilline, de l'amoxycilline, de l'épicilline, de la ticarcilline, de la cyclacilline, de la pirbénicilline, de l'azlocilline, de la mézlocilline, de la celbénicilline, l'ester acétoxyméthylique, pivaloyloxyméthylique, $\alpha$-éthoxycarbonyloxyéthlique ou phtalidylique d'ampicilline, de benzylpénicilline ou d'amoxycilline, de l'hétacilline, de la métampicilline, les $\alpha$-esters phényliques ou indanylique de carbénicilline ou de ticarcilline, de la céfatrizine, de la céphaloridine, de la céphalothine, de la céfazoline, de la céphalexine, du céphacétrile, du nafate de céphamandole, de la céphapirine, de la cépradine, de la 4-hydroxycéphalexine, du céphaparole ou de la céphaloglycine.

13. Composition suivant l'une quelconque des revendications 8 à 12, renfermant de l'amoxycilline trihydrate ou un sel pharmaceutiquement acceptable d'amoxycilline.

14. Composition suivant l'une quelconque des revendications 8 à 12, renfermant de la benzylpénicilline ou un sel pharmaceutiquement acceptable de benzylpénicilline.

15. Composition suivant l'une quelconque des revendications 8 à 12, renfermant de l'ampicilline ou un sel pharmaceutiquement acceptable d'ampicilline.

16. Composition suivant l'une quelconque des revendications 8 à 12, renfermant de la mézlocilline ou un sel pharmaceutiquement acceptable de mézlocilline.

17. Composition suivant l'une quelconque des revendications 8 à 12, renfermant de l'azlocilline ou un sel pharmaceutiquement acceptable d'azlocilline.

18. Composition suivant l'une quelconque des revendications 8 à 12, renfermant de la ticarcilline ou un sel pharmaceutiquement acceptable de ticarcilline.

19. Composition suivant l'une quelconque des revendications 8 à 12, renfermant de la phénoxyméthylpénicilline ou un sel pharmaceutiquement acceptable de celle-ci.

20. Composition suivant l'une quelconque des revendications 8 à 12, renfermant de céphaloridine.

21. Composition suivant l'une quelconque des revendications 8 à 12, renfermant de la céfazoline ou un sel pharmaceutiquement acceptable des céfazoline.

22. Composition suivant l'une quelconque des revendications 8 à 21, dans laquelle le rapport entre le composé de formule (II) et l'antibiotique à cycle $\beta$-lactame est compris entre 1:1 et 1:3.

23. Composition pharmaceutique renfermant de 150 à 1000 mg d'amoxycilline sous forme du trihydrate ou d'un sel pharmaceutiquement acceptable et de 25 à 500 mg d'acide 9-N-isobutylaminodésoxyclavulanique et un excipient pharmaceutiquement acceptable.

24. Composition pharmaceutique renfermant de 200 à 2000 mg de ticarcilline ou de ticarcilline disodique et de 75 à 250 mg d'acide 9-N-isobutylaminodésoxyclavulanique et un excipient pharmaceutiquement acceptable.

25. Composition suivant l'une quelconque des revendications 8 à 24, destinée à être utilisée pour le traitement d'infection bactérienne.

26. Acide 9-N-Isobutylaminodésoxyclavulanique destiné à être utilisé comme inhibiteur de $\beta$-lactamase ou comme agent antibactérien.

27. Procédé pour la préparation d'un composé suivant la revendication 1, ce procédé consistant à effectuer l'hydrogénation catalytique d'un composé de formule (V):

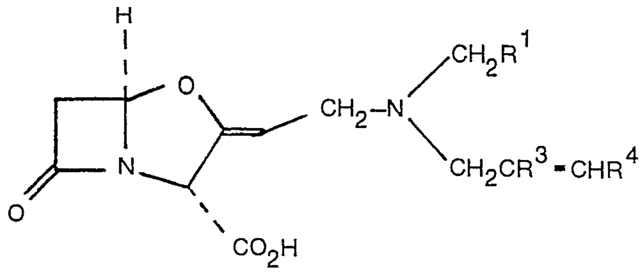

(V)

ou d'un ester de celui-ci, formule dans laquelle $R^1$ est un groupe isopropyle ou isopropylène, et $R^3$ et $R^4$ représentent ensemble une fraction molaire butadiène, puis si nécessaire à former un sel d'addition avec un acide d'un ester.

28. Procédé suivant la revendication 27, selon lequel le composé de formule (V) est sous la forme d'un ester hydrogénolysable.

29. Procédé suivant la revendication 28, selon lequel le composé de formule (V) est sous la forme d'un ester benzylique.

**Patentansprüche**

1. Die Verbindung der Formel (II):

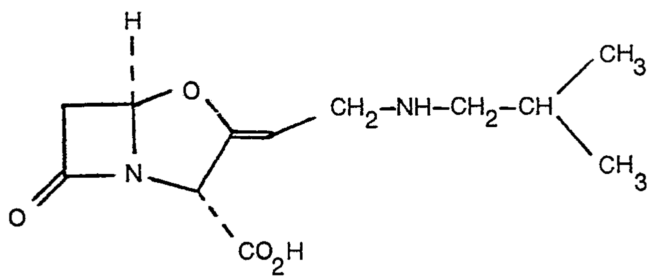

(II)

oder ein Ester oder ein Säureadditionssalz eines solchen Esters.

2. Eine Verbindung gemäß Anspruch 1, wenn sie in einer pharmakologisch verträglichen Form vorliegt.

3. 9-N-Isobutylaminodesoxyclavulansäure.

4. 9-N-Isobutylaminodesoxyclavulansäure, wenn sie in kristalliner Form vorliegt.

5. Eine Verbindung der Formel (III) oder (IV):

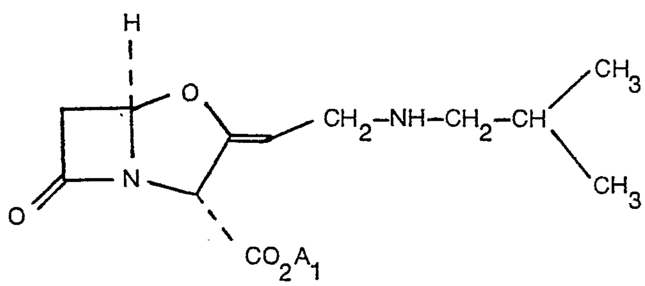

(III)

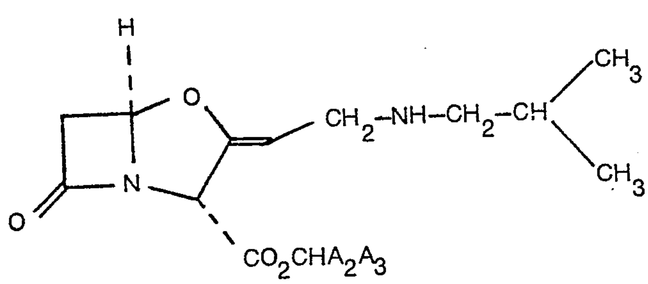

(IV)

in denen $A_1$ ein Alkylrest mit 1 bis 6 Kohlenstoffatomen ist, der gegebenenfalls mit einem Alkoxy- oder Acyloxyrest mit 1 bis 7 Kohlenstoffatomen substituiert ist; $A_2$ ein Alkenyl-oder Alkinylrest mit bis zu 5 Kohlenstoffatomen oder eine Phenyl-rest ist, der gegebenenfalls mit einem Fluor-, Chlor-, Bromatom, einer Nitrogruppe oder einem Alkyl- oder Alkoxyrest mit bis zu 4 Kohlenstoffatomen substituiert ist; und $A_3$ ein Wasserstoffatom, ein Alkylrest mit bis zu 4 Kohlenstoffatomen oder ein Phenylrest ist, der gegebenenfalls mit einem Fluor-, Chlor-, Bromatom, einer Nitrogruppe oder einem Alkyl- oder Alkoxyrest mit bis zu 4 Kohlenstoffatomen substituiert ist.

6. Eine Verbindung der Formel (IV) gemäß Anspruch 5, in der $A_2$ ein Phenyl-, p-Methoxyphenyl- oder p-Nitrophenylrest und $A_3$ ein Wasserstoffatom ist.

7. Eine Verbindung gemäß einem der Ansprüche 2,5 oder 6, wenn sie in Form eines Hydrochlorid-, Phosphat-, Sulfat-, Methan-sulfonat-, Toluolsulfonat-, Citrat-, Maleat-, Acetat-, Lactat-, Tartrat-, Propionat- oder Succinat-Säureadditionssalzes vorliegt.

8. Eine pharmazeutische Zubereitung, die eine Verbindung gemäß einem der Ansprüche 2 bis 7 und einen pharmakologisch verträglichen Träger enthält.

9. Eine pharmazeutische Zubereitung gemäß Anspruch 8, wobei die Verbindung der Formel (II) in kristalliner Form für die Verabreichung durch Injektion oder Infusion angepaßt ist.

10. Eine pharmazeutische Zubereitung gemäß Anspruch 8 oder 9, wobei eine injizierbare wäßrige Lösung nicht weniger als 15 Gewichts-prozent der Verbindung der Formel (II) enthält.

11. Eine Zubereitung gemäß einem der Ansprüche 8 bis 10, die auch ein $\beta$-Lactam-Antibiotikum enthält.

12. Eine Zubereitung gemäß Anspruch 11, die Benzylpenicillin, Phenoxymethylpenicillin, Carbenicillin, Azidocillin, Propicillin, Ampicillin, Amoxycillin, Epicillin, Ticarcillin, Cyclacillin, Pirbenicillin, Azlocillin, Mezlocillin, Celbenicillin, den Acetoxymethyl-, Pivaloyloxymethyl-, $\alpha$-Äthoxy-carbonyloxyäthyl- oder Phtalidylester von Ampicillin, Benzylpenicillin oder Amoxycillin, Hetacillin, Metampicillin, die Phenyl- oder Indanyl-$\alpha$-Ester von Carbenicillin oder Ticarcillin, Cefratrizin, Cephaloridin, Cephalotin, Cefazolin, Cefalexin, Cephacetril, Cephamandol- nafat, Cephapirin, Cepradin, 4-hydroxycephalexin, Cefaparol oder Cephaloglycin enthält.

13. Eine Zubereitung gemäß einem der Ansprüche 8 bis 12, die Amoxycillin-trihydrat oder ein pharmakologisch verträgliches Salz von Amoxycillin enthält.

14. Eine Zubereitung gemäß einem der Ansprüche 8 bis 12, die Benzylpenicillin oder ein pharmakologisch verträgliches Salz von Benzylpenicillin enthält.

15. Eine Zubereitung gemäß einem der Ansprüche 8 bis 12, die Ampicillin oder ein pharmakologisch verträgliches Salz von Ampicillin enthält.

16. Eine Zubereitung gemäß einem der Ansprüche 8 bis 12, die Mezlocillin oder ein pharmakogolisch verträgliches Salz von Mezlocillin enthält.

17. Eine Zubereitung gemäß einem der Ansprüche 8 bis 12, die Azlocillin oder ein pharmakologisch verträgliches Salz von Azlocillin enthält.

18. Eine Zubereitung gemäß einem der Ansprüche 8 bis 12, die Ticarcillin oder ein pharmakologisch verträgliches Salz von Ticarcillin enthält.

19. Eine Zubereitung gemäß einem der Ansprüche 8 bis 12 die Phenoxymethylpenicillin oder ein pharmakologisch verträgliches Salz davon enthält.

20. Eine Zubereitung gemäß einem der Ansprüche 8 bis 12, die Cephaloridin enthält.

21. Eine Zubereitung gemäß der Ansprüche 8 bis 12, die Cefazolin oder ein pharmakologisch verträgliches Salz von Cefazolin enthält.

22. Eine Zubereitung gemäß einem der Ansprüche 8 bis 21, in der das Verhältnis der Verbindung der Formel (II) zum $\beta$-Lactam-Antibiotikum 1:1 bis 1:3 beträgt.

23. Eine pharmazeutische Zubereitung, die von 150 bis 1000 mg Amoxycillin als Trihydrat oder pharmakologisch verträgliches Salz und von 25 bis 500 mg 9-N-Isobutylaminodesoxyclavulansäure und einen pharmakologisch verträglichen Träger enthält.

24. eine pharmazeutische Zubereitung, die 200 bis 2000 mg Ticarcillin oder Di-Natrium-ticarcillin und von 75 bis 250 mg 9-N-Isobutylaminodesoxyclavulansäure und einen pharmakologisch verträglichen Träger enthält.

25. Eine Zubereitung gemäß einem der Ansprüche 8 bis 24, zur Verwendung in der Behandlung von bakteriellen Infektionen.

26. 9-N-Isobutylaminodesoxyclavulansäure für die Verwendung als $\beta$-Lactamase-Inhibitor oder als antibakterielles Mittel.

27. Ein Verfahren zur Herstellung einer Verbindung gemäß Anspruch 1, die Maßnahmen umfassend, daß eine Verbindung der Formel (V):

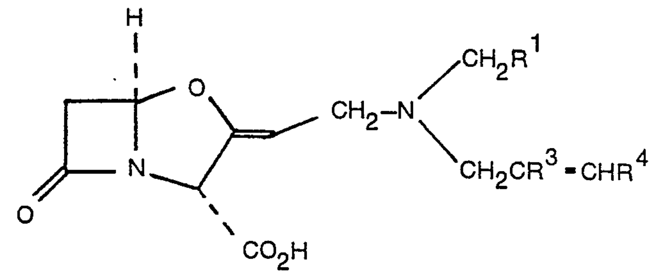

(V)

oder ein Ester derselben, wobei $R^1$ ein Isopropyl- oder Isopropylenrest ist, und $R^3$ und $R^4$ zusammen einen Butadien-Rest darstellen, katalytisch hydriert wird, und anschließend gegebenenfalls ein Säureadditionssalz eines Esters gebildet wird.

28. Verfahren gemäß Anspruch 27, in dem die Verbindung der Formel (V) in Form eines hydrierbaren Esters ist.

29. Verfahren gemäß Anspruch 28, in dem die Verbindung der Formel (V) in Form eines Benzylesters ist.